# EUROPEAN PATENT SPECIFICATION

(11) **EP 2 812 688 B1**
(45) Date of publication and mention of the grant of the patent: **12.09.2018**
(21) Application number: 12813865.8
(22) Date of filing: 21.12.2012
(51) Int. Cl.: G01N 33/50

(54) **MAO-B INHIBITORS DERIVED FROM THE ONE-CARBON CYCLE**
AUS DEM EINZELKOHLENSTOFFZYKLUS GEWONNENE MAO-B-INHIBITOREN
INHIBITEURS DE MAO-B ISSUS DU CYCLE DU CARBONE SIMPLE

(30) Priority: 23.12.2011 GB 201122227
(43) Date of publication of application: 17.12.2014
(73) Proprietor: Randox Laboratories Ltd., Crumlin, County Antrim BT29 4QY (GB)
(72) Inventor: ZELLNER, Maria, 1090 Wien (AT); UMLAUF, Ellen, 1090 Wien (AT)
(86) International application number: PCT/EP2012/076815
(87) International publication number: WO 2013/093079

(56) References cited:
- US-A1- 2009 197 824
- US-A1- 2011 067 123
- J. C. SHIH ET AL: "Regulation of MAO-A and MAO-B Gene Expression", CURRENT MEDICINAL CHEMISTRY, vol. 11, no. 15, 1 August 2004 (2004-08-01), pages 1995-2005, XP055056720, ISSN: 0929-8673, DOI: 10.2174/0929867043364757
- SMITH A DAVID ET AL: "Homocysteine-Lowering by B Vitamins Slows the Rate of Accelerated Brain Atrophy in Mild Cognitive Impairment: A Randomized Controlled Trial", PLOS ONE, PUBLIC LIBRARY OF SCIENCE, US, vol. 5, no. 9, 1 September 2010 (2010-09-01), pages e12244-1, XP009141832, ISSN: 1932-6203
- MARIA ZELLNER ET AL: "A proteomics study reveals a predominant change in MaoB expression in platelets of healthy volunteers after high protein meat diet: relationship to the methylation cycle", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 118, no. 5, 20 March 2011 (2011-03-20), pages 653-662, XP019901436, ISSN: 1435-1463, DOI: 10.1007/S00702-011-0617-6
- PARNETTI, L., ET AL: "Platelet MAO-B Activity and Vitamin B12 in Old Age Dementias", MOLECULAR AND CHEMICAL NEUROPATHOLOGY, vol. 16, 1992, pages 23-32, XP009168005,

## Description

### BACKGROUND

### Alzheimer's disease

Late-onset Alzheimer's disease (LOAD) is the most prominent form of dementia among the elderly (> 65 yrs). The only known genetic risk factor is the Apolipprotein E4 allele. In contrast to the early-onset variant, LOAD patients do not carry any mutations in the genes of amyloid precursor protein (APP), presenilin 1 and presenilin 2. The disease is characterized by progressive cognitive dysfunction, and various behavioural and neuro-psychiatric disturbances such as agitation, psychosis, depression, apathy, disinhibition, anxiety, purposeless behaviour, and disorders of sleep and appetite. Histopathological hallmarks in the brain are extracellular amyloid plaques and neurofibrillary tangles in neurons. Amyloid plaques are formed by aggregation of amyloid beta (1-42) that is derived from proteolytic processing of APP by beta and gamma secretases. Neurofibrillary tangles are found in cell bodies and apical dendrites as neurofibrillary tangles, in distal dendrites as neuropil threads, and in the abnormal neurites that are associated with some plaques (neuritic plaques).They are mainly composed of hyperphosphorylated aggregates of the protein tau.

### DNA methylation and Alzheimer's disease

Approximately 70% of annotated gene promoters are associated with CpG islands, including most of the housekeeping genes, many tissue-specific genes and developmental regulator genes. CpG islands are sites of transcriptional initiation and CpG island methylation leads to silencing of the associated promoter, and to decreased expression levels of the respective protein. Global DNA hypomethylation has been observed in the entorhinal cortex of LOAD patients. It has also been shown that several proteins such as presenilin 1 and APP that are involved in the beta amyloid formation in LOAD are regulated by promoter methylation. Additionally, APOE, the only known genetic LOAD-risk factor, and genes involved in methylation homeostasis (MTHFR, DNMT1) show a significant inter-individual epigenetic variability in LOAD, which may contribute to LOAD predisposition.

### The one-carbon cycle methylation by SAM

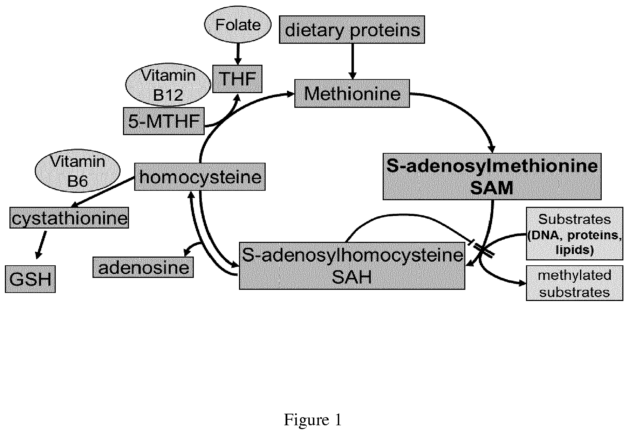

DNA methyltransferases (DNMTs) use S-adenosylmethionine (SAM), the main methyl donor in eukaryotes, as substrate for methylating cytosines on genomic DNA. The resulting S-adenosylhomocysteine (SAH) is a competitive inhibitor of DNMTs. SAH is hydrolyzed to adenosine and homocysteine, which can be re-methylated to methionine. These compounds are connected via the One-carbon cycle which is the only means of SAM production (Figure 1).

### The one-carbon cycle and Alzheimer's disease

Administration of folic acid, vitamin B12 and vitamin B6 to AD patients with mild cognitive impairment for two years reduced brain atrophy and improved cognitive function when compared to the placebo group (1). Vitamin B12 therapy significantly reduced platelet MAO activity (Mao-B is the predominant isoform in platelets) in LOAD patients to apparently normal levels (2). The Inventors showed that increased meat consumption improves cognitive functions in young volunteers and reduces the platelet Mao-B level and that platelet Mao-B concentration inversely correlates with the plasma level of vitamin B12, which is a cofactor in the One-carbon cycle (3).

### MAO-B, MAO-B inhibitors, Alzheimer's disease and other pathologies

The flavoenzyme monoamine oxidase B (MAO-B) is located in the outer membrane of mitochondria and catalyses the oxidative deamination of amine neurotransmitters and dietary amines using O₂ as the electron acceptor. MAO-B preferentially degrades phenylethylamine and benzylamine, as well as the cognition enhancing neurotransmitter dopamine. Oxidative deamination of primary monoamines by MAO-B results in the species with proven or potential toxicity, namely ammonia, aldehydes and hydrogen peroxide. Furthermore, MAO-B inhibition reduces dopamine inactivation enabling the neurotransmitter's increased availability in dopaminergic neurons. The activity of MAO-B in brain and in platelets is significantly increased in LOAD (EP1891445) and it has been demonstrated that MAO-B mRNA and MAO-B protein levels are elevated in platelets of LOAD patients (PCT/GB2010/052023). MAO-B inhibitor drugs currently find use in the treatment of hypertension, Parkinson's disease, senile dementia and depression. Thus evidence suggests the potential use of MAO-B inhibitors for the treatment of AD.

The search for therapeutic drugs for the treatment of debilitating pathologies is an ongoing and relentless process. However, it is also a lengthy and extremely costly exercise, with most candidate drug development programs being terminated before drug approval. The elucidation of possible pathophysiological pathways is an extremely difficult but highly desirable goal as it enables a more targeted approach to candidate drug choice, expedites the drug development process and increases the chances of a drug being approved for therapy. Diseases such as hypertension, depression, Parkinson's disease and dementias can be treated using MAO-B inhibitors. Described herein are novel compounds of the MAO-B inhibitor class identified through the elucidation of the connection of MAO-B to the One-carbon cycle.

### References

(1) Smith A.D. et al. (2010). PLoS One, 8;5(9): e12244.
(2) Regland B. et al. (1991). Eur. Arch. Psychiatry Clin. Neurosci., 240(4-5): 288-91.
(3) Zellner M. et al. (2011). J Neural Transm., 118(5): 653-62.

### Figures

Figure 1 One-carbon cycle of SAM production

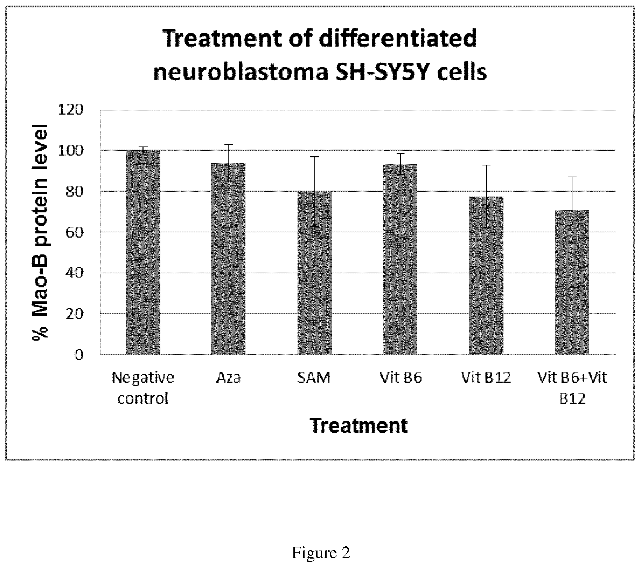

Figure 2 Relative % MAO-B levels in cells treated with SAM, vitamin 6, vitamin 12 and control
Figure 3 Results of experiment treating Hep2G cells with 5-aza-2-deoxycytidine in relation to MAO-B expression levels normalised against GAPDH and 14-3-3 gamma.
Figure 4 Results of experiment treating Hep2G cells with 5-aza-2-deoxycytidine in relation to presenilin 1 expression levels normalised against GAPDH and 14-3-3 gamma.
Figure 5 Results of experiment treating Hep2G cells with vitamins B12 & B6 in relation to MAO-B expression levels normalised against GAPDH and 14-3-3 gamma.
Figure 6 Results of experiment treating Hep2G cells with 5-aza-2-deoxycytidine in relation to presenilin 1 expression levels normalised against GAPDH and 14-3-3 gamma.

### SUMMARY OF THE INVENTION

The invention, underpinned by the finding that the concentration of MAO-B in patient samples is affected by the SAM methylation cycle, describes the utility of SAM and compounds in the One-carbon cycle in disease treatment therapies in which the disease treatment depends upon MAO-B inhibition. Elucidation of the relationship between MAO-B concentrations and the components of the SAM one-carbon methylation cycle also enables methods of measuring the efficacy of a drug of or a drug that affects the SAM one-carbon methylation cycle in *in vitro* samples taken from patients, especially patients suspected of having or suffering from Alzheimer's Disease, especially late onset Alzheimer's disease.

### DETAILED DESCRIPTION OF THE INVENTION

In a first aspect, the disclosure describes the use of one or more of the compounds SAM, vitamin B6, vitamin B12, and folate, individually or in any combination, in the treatment or prevention of diseases which are mediated by monoamine oxidase B inhibitors. In a preferred embodiment the compound(s) is/are one or more of SAM, vitamin B6 and B12; SAM is an especially preferred embodiment. A combination of vitamin B6 and B12 is also a preferred embodiment.

A second aspect of the disclosure is the use as adjunct therapy of one or more of the compounds SAM, vitamin B6, vitamin B12 and folate, individually or in any combination, in the treatment and prevention of diseases which are mediated by monoamine oxidase B inhibitors. A preferred disease to be treated or prevented by the compounds of the invention when used as adjunct therapy is Alzheimer's disease; treatment of late onset Alzheimer's disease is an especially preferred embodiment. Adjunct therapy implies that the one or more compounds can be used in conjunction with other recognised forms of therapy for a specified diseased.

A preferred disease to be treated or prevented by the compounds of the disclosure whether alone or as adjunct therapy is Alzheimer's disease; treatment of late onset Alzheimer's disease is an especially preferred embodiment.

A further aspect of the disclosure is a method of determining the efficacy of a drug or drugs of the SAM one-carbon methylation cycle for use in therapy comprising measuring in an *in vitro* sample taken from a patient or from cultured or harvested cells the concentration of MAO-B following administration of a drug or drugs of the SAM one-carbon methylation cycle to the patient or to the cultured or harvested cells and comparing said concentration to a control concentration of MAO-B. The control MAO-B concentration can be a statistical measure of central tendency such as the median or mean of a suitable patient population. Preferably, the control concentration of MAO-B is the concentration of MAO-B in an *in vitro* sample taken from the patient or from cultured or harvested cells before administration of a drug or drugs. In an embodiment of the disclosure the MAO-B concentrations measured are normalised using a suitable compound such as GAPDH or 14-3-3 gamma. This aids in minimising the effect of differences in the inter-sample and inter-cellular MAO-B concentrations and can increase result accuracy. In a preferred embodiment the method is used to test the efficacy of drugs of the SAM one-carbon methylation cycle that are used or are to be used in Alzheimer's disease therapy, especially late onset Alzheimer's disease. A concentration of MAO-B in an *in vitro* sample taken from the patient or from cultured or harvested cells following drug or drugs administration that is lower than that of the control is indicative of an efficacious drug or drugs. What is meant by drug(s) of the SAM one-carbon methylation cycle is a chemical compound that is mentioned in Figure 1. Representative drugs of the SAM one-carbon methylation cycle include either as monotherapy or in combination: SAM, folate, vitamin B12 and vitamin B6. In a preferred embodiment the drug or drugs of the SAM one-carbon methylation cycle is/are SAM, vitamin B6 and vitamin B12. Most preferably the monotherapeutic drug is SAM, vitamin B6 or vitamin B12 and the combination of drugs is vitamin B6 + vitamin B12. If the concentration of MAO-B measured in the *in vitro* sample taken from the patient or in the cultured or harvested cells is reduced, this denotes that the drug or drugs of the SAM one-carbon methylation cycle is working i.e. the drug(s) is efficacious and can be used for the treatment of AD, preferably LOAD. The skilled person would acknowledge the possible use of numerous cell types for testing drug efficacy; preferred cultured or harvested cells of the method of the disclosure are HepG2 cells or differentiated neuroblastoma SH-SY5Y cells. The compounds of the SAM one-carbon methylation cycle for all aspects of the disclosure can be in the form of pharmaceutically acceptable salts.

Alternatively, for pathologies in which an increase in MAO-B concentration is sought, it might be clinically desirable to associate the SAM one-carbon methylation cycle with an increase in MAO-B concentration in a sample taken from a patient. An increase in MAO-B would be expected if the SAM one-carbon cycle were to be interrupted by a drug or drugs causing a decrease in DNA-methylation. A further embodiment of the disclosure is a method of determining the efficacy of a drug or drugs in a patient to inhibit the SAM one-carbon methylation cycle comprising measuring in an *in vitro* sample taken from the patient or from cultured or harvested cells the concentration of MAO-B following administration of a drug or drugs to the patient or to the cultured or harvested cells and comparing said concentration to a control concentration of MAO-B. An increase in the concentration of MAO-B in the *in vitro* patient sample or the cultured or harvested cells compared to control indicates that the SAM one-carbon cycle has been inhibited and that the drug or drugs is/are efficacious and achieve the desired effect. The invention is a method of determining the effect of S-adenosylmethionine or vitamin B6 plus vitamin B12 upon the SAM one-carbon methylation cycle comprising
i. taking cultured or harvested cells
ii. adding S-adenosylmethionine or vitamin B6 plus vitamin B12 to the cultured or harvested cells
iii. measuring the MAO-B concentration in the cultured or harvested cells
iv. comparing the concentration of MAO-B measured in iii. to a control concentration of MAO-B
in which a lower MAO-B concentration in the cultured or harvested cells after addition of S-adenosylmethionine or vitamin B6 plus vitamin B12 compared to the control concentration indicates promotion of the SAM one-carbon methylation cycle, while a greater MAO-B concentration indicates inhibition of the SAM one-carbon methylation cycle. The lower or greater MAO-B concentration of treated cells following addition of compounds compared to control is an indirect measure of the degree of DNA-methylation, a greater concentration indicating promotion of DNA methylation. The methods described herein make use of the link that has been elucidated between the SAM one-carbon methylation cycle and the MAO-B concentration and support the evaluation of the efficacy and usefulness of current and future experimental and therapeutic drugs and the use of certain compounds of the SAM one-carbon methylation cycle for treating AD, especially LOAD.

### Methods and Results

### Example 1

The neuroblastoma cell line SH-SY5Y was seeded at 110,000 cells/6-well. Neuronal differentiation treatment was started on the next day (=Day 0) by addition of 10 µM retinoic acid every second day until Day 6. On Day 8 differentiation treatment was switched to 5 µM uridine (Ur), 5 µM 5-fluoro-2'-deoxyuridine FdUr), and 1 µM cytosine beta-D-arabinofuranoside (araC). During mitotic inhibitor addition (= Days 8 to 12) cells were left untreated or treated with 625 nM 5-aza-2'-deoxycytidine (Aza), 25 µM S-adenosylmethionine (SAM), 5 µg/ ml vitamin B6 (Vit B6), 7.5 µg/ml vitamin B12 (Vit B12), or 5 µg/ ml vitamin B6 / 7.5 µg/ml vitamin B12. On Day 12 cells were harvested. Cells were washed twice with chilled PBS, scraped off in PBS/protease inhibitors, and centrifuged at 15000 x g at 4C for 10 min. The pellet was reconstituted in 150 µl PBS/protease inhibitors and precipitated with 50 µl trichloroacetic acid/80 mMDTT for 1 hour at 4C. After centrifugation at 15000 x g at 4C for 10 min, precipitated protein was washed four times with 0.6 ml acetone/20 mM DTT and stored at -80C until analysis. The dried total protein pellet was reconstituted in 7M urea/2M thiourea/4%CHAPS buffer and the protein concentration was determined using the Coomassie Plus (Bradford) Protein Assay (Pierce, Thermo Scientific). Equal protein amounts were loaded onto an 11% SDS polyacrylamide gel and proteins were blotted onto nitrocellulose. Mao-B was detected using a polyclonal Goat anti-Mao-B antibody (sc18401, Santa Cruz) and DyLight 649 AffiniPureF(ab')2 Fragment Donkey Anti-Goat IgGgamma (Jackson ImmunoResearch Laboratories, Inc.), and a Typhoon scanner (GE Healthcare Life Sciences). Results were analysed with the software ImageJ. The signals of the Mao-B bands of SAM treated and untreated differentiated SH-SY5Y cells were compared and the latter was set 100%.

### Example 2

### Treatment with 5-aza-2-deoxycytidine.

HepG2 cells were grown in DMEM low glucose/10%FBS and were treated with 10 µM 5-aza-2-deoxycytidine for 1, 2, or 3 days. Untreated HepG2 cells served as controls. Cells were scraped in precipitation buffer (PBS/25% trichloroacetic acid/20 mM DTT/ protease inhibitors) at 4C and proteins were precipitated for 1 hour at 4C. Precipitated proteins were pelleted at 15000 rpm for 10 min (4C) and washed 4 times with 700 µl acetone/20 mM DTT. Before analysis by SDS PAGE and Western blot, the precipitated samples were reconstituted in DIGE buffer/1% pH4-7 ampholyte. Six parallel experiments were performed. Each sample was run on two different Western blots and the results are displayed as averages. Two normalisation compounds were used: GAPDH (glyceraldehyde 3-phosphate dehydrogenase) and 14-3-3 gamma (protein kinase C inhibitor protein 1). Each Mao-B band signal was divided by the respective GAPDH or 14-3-3 gamma band signal to achieve normalisation. As band signals on different Western blots were not comparable due to the use of different antibody solutions, all normalised Mao-B signals of treated cells were compared to the normalised Mao-B signals of the untreated cells, which were set at 100%.

### Treatment with vitamins B6 and B12

HepG2 cells were grown in DMEM low glucose/2%FBS and were treated with vitamin B6 (300 µg/ml) and vitamin B12 (74.4 µg/ml) for 1, 3, 4, 5 and 7 days. Untreated HepG2 cells served as controls. Cells were harvested and analysed by Western blot.

### Results

**Table 1:** Summary of three experiments: SH-SY5Y cells were differentiated with retinoic acid and mitotic inhibitors. Cells were treated with different agents, harvested, and Mao-B protein levels were analysed by Western blot. For each experiment the Mao-B signals of treated SH-SY5Y cells were compared to untreated cells (%Mao-B of untreated cells).

| | **Densitometric quantification** | | | **% MAO-B of untreated cells** | | | |
|---|---|---|---|---|---|---|---|
| **Treatment** | **Exp. 1** | **Exp. 2** | **Exp. 3** | **Exp. 1** | **Exp. 2** | **Exp. 3** | **Average** |
| Control | 4113.8 | 1136.2 | 1093.1 | 100 | 102 | 98 | 100 |
| Aza | - | 1116.9 | 973.5 | - | 100 | 87 | 94 |
| SAM | 2482.6 | 990.4 | 1006.8 | 60 | 89 | 90 | 80 |
| Vit B6 | 4087.1 | 1008.1 | 1004.8 | 99 | 90 | 90 | 93 |
| Vit B12 | 3823.5 | 857.8 | 695.3 | 93 | 77 | 62 | 77 |
| Vit B6 & B12 | 3425.1 | 852.2 | 585.8 | 83 | 76 | 53 | 71 |

As can be seen from Table 1, SAM, vitamin B6, vitamin B12 and a combination of vitamin B6 and B12 all resulted in a substantial decrease in MAO-B levels compared to the negative control. This suggests a link between the methyl donor S-adenosylmethionine (SAM) and the Mao-B protein expression level via the One-carbon cycle, DNA methyltransferases and MAOB promoter methylation. Increased availability of SAM, a methyl donor for DNA methyltransferases, may result in elevated MAOB promoter methylation and a decrease in transcription and protein expression of the Mao-B protein. Therefore, SAM appears to regulate Mao-B activity and could be used in therapies of late-onset Alzheimer's disease, Parkinson's disease and other diseases mediated by MAO-B inhibitors. Furthermore, as indicated by the results in Table 1, other compounds of the One-carbon cycle (Figure 1) are also able to indirectly affect MAO-B levels.

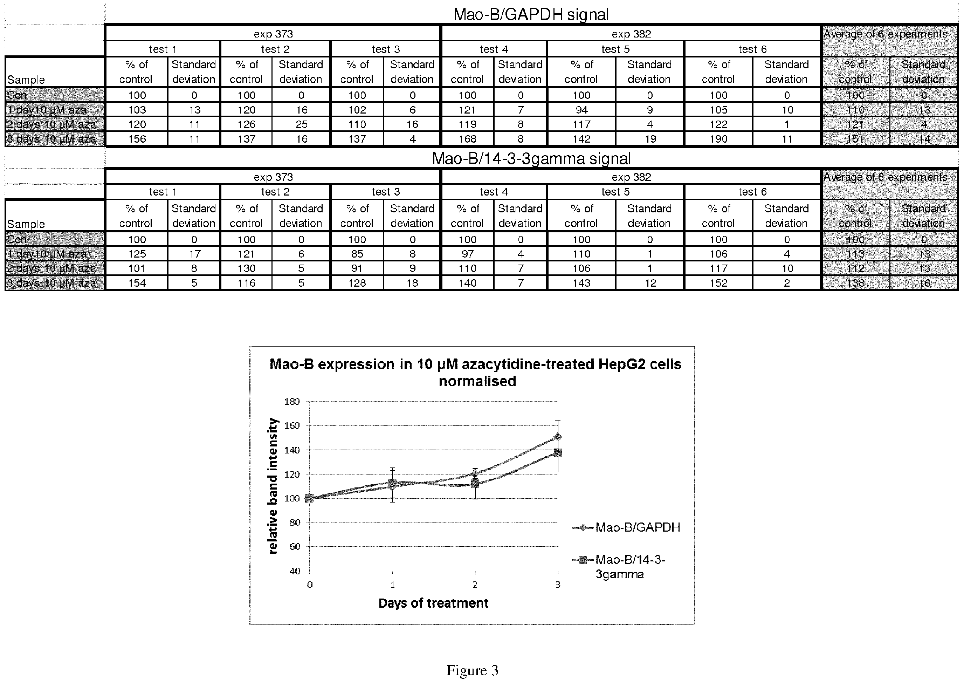

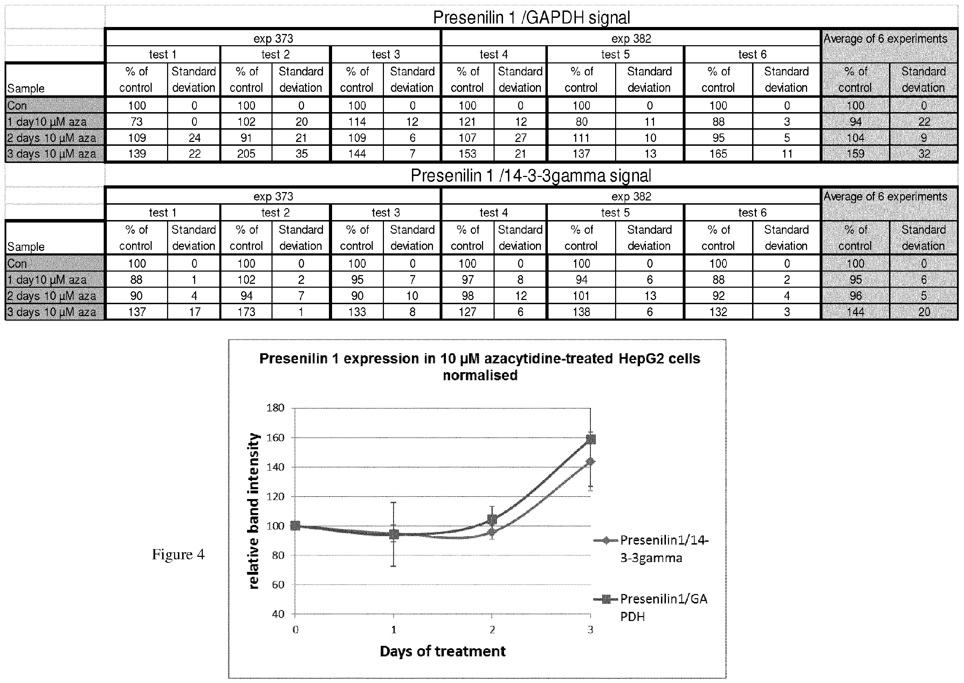

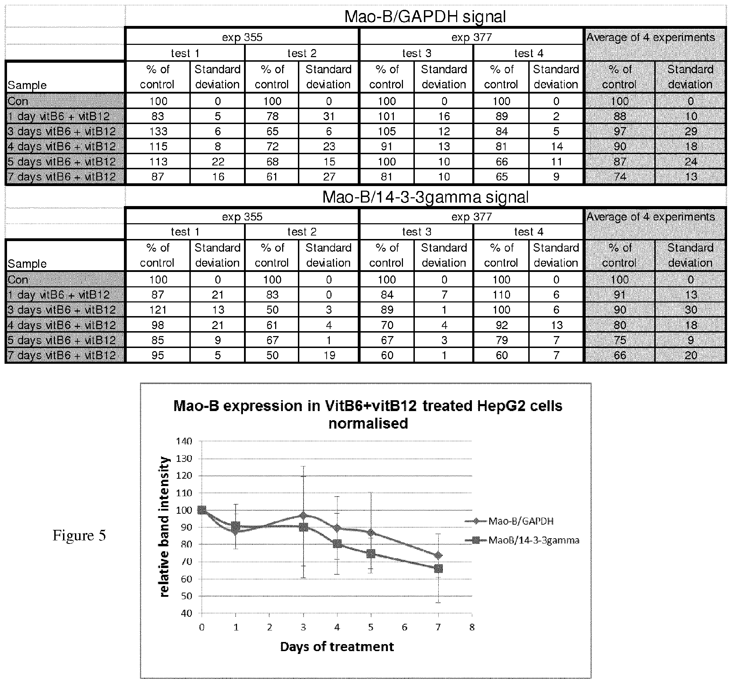

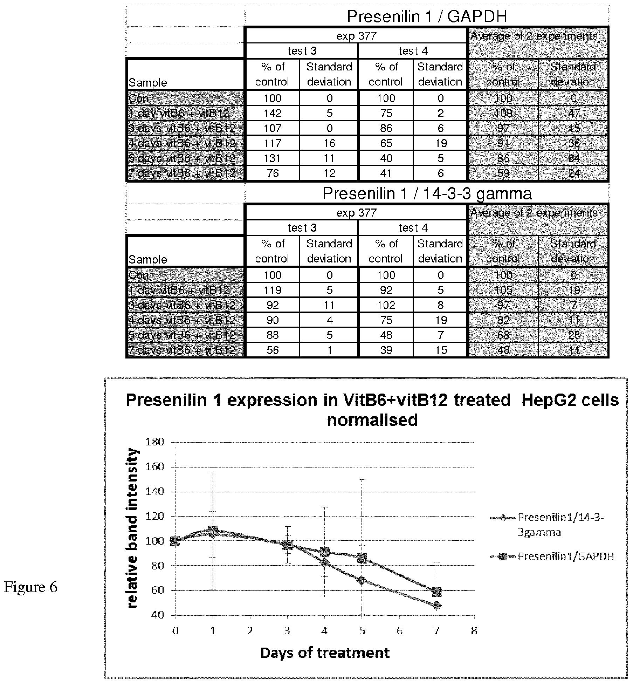

Figures 3 to 6 display data that consolidates the physiological connection between the SAM one-carbon cycle and MAO-B levels. Figure 3 displays the effect of the DNA methyltransferase inhibitor 5-aza-2-deoxycytidine on MAO-B protein expression in HepG2 cells. This data suggests that due to a reduced level of CpG island methylation, increased levels of MAO-B are expressed. Figure 5 displays the effect of a combination of vitamins B12 & 6 in down-regulating MAO-B protein expression by increasing the supply of SAM and subsequent CpG island methylation increases. Figures 4 and 6 display the corresponding effects of 5-aza-2-deoxycytidine and vitamins B12 & B6 on the expression of the AD-related protein preselinin 1; the result of these positive control experiments support the validity of the MAO-B expression experiments and results.

## Claims

1. A method of determining the effect of S-adenosylmethionine or vitamin B6 plus vitamin B12 upon the S-adenosylmethionine one-carbon methylation cycle comprising
i. taking cultured or harvested cells
ii. adding S-adenosylmethionine or vitamin B6 plus vitamin B12 to the cultured or harvested cells
iii. measuring the monoamine oxidase-B concentration in the cultured or harvested cells
iv. comparing the concentration of monoamine oxidase-B measured in iii. to a control concentration of monoamine oxidase-B
in which a lower monoamine oxidase-B concentration in the cultured or harvested cells after addition of S-adenosylmethionine or vitamin B6 plus vitamin B12 compared to the control concentration indicates promotion of the S-adenosylmethionine one-carbon methylation cycle, while a greater monoamine oxidase-B concentration indicates inhibition of the S-adenosylmethionine one-carbon methylation cycle.

## Patentansprüche

1. Verfahren zur Bestimmung der Wirkung von S-Adenosylmethionin oder Vitamin B6 plus Vitamin B12 auf den Ein-Kohlenstoff-Methylierungszyklus von S-Adenosylmethionin umfassend
i. Aufnahme kultivierter oder geernteter Zellen
ii. Zugabe von S-Adenosylmethionin oder Vitamin B6 plus Vitamin B12 zu den gezüchteten oder geernteten Zellen
iii. Messung der Monoaminoxidase-B Konzentration in den gezüchteten oder geernteten Zellen
iv. Vergleichen der Konzentration von Monoaminoxidase-B, gemessen in iii. zu einer Kontrollkonzentration von Monoaminoxidase-B
in denen eine niedrigere Monoaminoxidase-B Konzentration in den kultivierten oder geernteten Zellen nach Zugabe von S-Adenosylmethionin oder Vitamin B6 plus Vitamin B12 im Vergleich zur Kontrollkonzentration eine Förderung des S-Adenosylmethionin-Ein-Kohlenstoff-Methylierungszyklus anzeigt, während eine größere Monoaminoxidase-B Konzentration eine Hemmung des Methylierungszyklus von S-Adenosylmethionin-Ein-Kohlenstoff-Methylierung anzeigt.

## Revendications

1. Procédé de détermination de l'effet de la S-adénosylméthionine ou de la vitamine B6 plus la vitamine B12 sur le cycle de méthylation mono-carbone de la S-adénosylméthionine comprenant
i. prendre des cellules cultivées ou récoltées
ii. l'addition de S-adénosylméthionine ou de vitamine B6 et de vitamine B12 aux cellules cultivées ou récoltées
iii. mesurer la concentration de monoamine oxydase-B dans les cellules cultivées ou récoltées
iv. comparer la concentration en monoamine oxydase-B mesurée en iii. à une concentration de contrôle de monoamine oxydase-B
dans lequel une concentration inférieure en monoamine oxydase-B dans les cellules cultivées ou récoltées après addition de S-adénosylméthionine ou de vitamine B6 plus vitamine B12 par rapport à la concentration de contrôle indique une promotion du cycle de méthylation mono-carbone de la S-adénosylméthionine, tandis qu'une plus grande la concentration en monoamine oxydase-B indique l'inhibition du cycle de méthylation mono-carbone de la S-adénosylméthionine.
